(19) Europäisches Patentamt
European Patent Office
Office européen des brevets

(11) Publication number: **0 476 408 A1**

(12) **EUROPEAN PATENT APPLICATION**

(21) Application number: **91114725.4**

(22) Date of filing: **02.09.91**

(51) Int. Cl.5: **A61K 47/48**, A61K 39/395, //A61K31/71,A61K31/535

(30) Priority: **17.09.90 US 583815**

(43) Date of publication of application: **25.03.92 Bulletin 92/13**

(84) Designated Contracting States: **CH DE FR GB IT LI SE**

(71) Applicant: **BRUNSWICK CORPORATION**
**One Brunswick Plaza**
**Skokie Illinois 60077(US)**

(72) Inventor: **Wrasidlo, Wolfgang A.**
**307 Prospect Street**
**La Jolla, California 92037(US)**

(74) Representative: **UEXKÜLL & STOLBERG**
**Patentanwälte**
**Beselerstrasse 4**
**W-2000 Hamburg 52(DE)**

# (54) Chemical conjugation of morpholino anthracyclines to antibodies.

(57) A morpholino anthracycline-linker compound comprising the structure M-L, wherein M is a morpholino anthracycline compound and L is a linker molecule which is attached to the carbonyl moiety on the $C_{13}$ substituent of the cyclohexane ring of M.

Figure 1

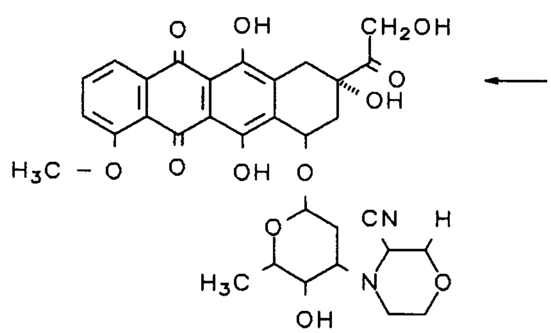

EP 0 476 408 A1

## BACKGROUND OF THE INVENTION

This invention generally relates to methods of synthesizing novel morpholino anthracycline derivatives and, more particularly, to methods of coupling morpholino anthracyclines and derivatives thereof to antibodies for use as therapeutic agents.

It has long been recognized as highly desirable to specifically target therapeutic agents to invading organisms or diseased cells. Specific targeting allows lower doses of the therapeutic agent to be administered and reduces the observed non-specific side effects. Various carriers such as liposomes, proteins, hormones, growth factors and antibodies have been used to specifically target therapeutic agents.

Antibodies, particularly monoclonal antibodies, which specifically recognize selected antigens are especially suited for targeting therapeutic agents. Monoclonal antibodies are advantageous since they have the ability to recognize a single molecular site or epitope on a cell. Studies have identified monoclonal antibodies specifically directed toward tumor-associated antigens and other antigens on cancer cells, T-cells and B-cells. These monoclonal antibodies can be used to deliver drugs directly and specifically to the target cells by first attaching the drug to the antibody. Delivery of such antibody-therapeutic agents to specific cells, tissues, organs, or any other sites in vivo can be accomplished using whole antibodies or fragments of antibodies. Such fragments should retain the ability to recognize selected antigens.

Antibody-therapeutic agent conjugates can be made by chemically coupling the two molecules through covalent bonds. The critical features of the resulting conjugate are that both the antibody and cytotoxic agent maintain their biological activity. Further, the attachment of the therapeutic agent to the target cell binding protein must be stable to all conditions of administration to a patient and under all conditions present in the microenvironment at the site of action. Moreover, for administration of an effective amount in a human or animal, the conjugate must remain immunospecific for an antigenic determinant on specific cells or tissues.

Morpholino anthracyclines are potent cytotoxic agents. Their potency is attributed to the combined effects of several factors. First, morpholino anthracyclines are potent inhibitors of DNA synthesis because of their ability to inhibit DNA topoisomerase II. Second, morpholino anthracyclines also inhibit RNA synthesis. This phenomenon is mostly attributed to the intercalating nature of the drug into the large groove of DNA and thereby impeding RNA polymerase movement. Finally, the morpholino substituent on these drugs greatly increases the lipophilicity, and therefore their ability to enter the cell, without changing the N-basicity. N-basicity is important for electrostatic binding of the drug to the phosphate backbone. Despite the combined cytotoxic effects of such factors, morpholino anthracyclines have not been attached to carrier molecules for the purpose of targeting specific cells. Attempts to attach particular cyanomorpholino anthracycline derivatives to carrier molecules have resulted in decomposition reactions.

There thus exists a need for a morpholino anthracycline antibody conjugate and a method for synthesizing such a conjugate, that is effective and useful for therapeutic or diagnostic purposes. The present invention satisfies these needs and provides related advantages as well.

## SUMMARY OF THE INVENTION

A morpholino anthracycline-linker compound comprising the structure M-L, wherein M is a morpholino anthracycline compound and L is a linker molecule which is attached to the carbonyl moiety on the $C_{13}$ substituent of the terminal cyclohexane ring of M.

## BRIEF DESCRIPTION OF THE FIGURES

Figure 1 shows the structure of a morpholino anthracycline compound, cyanomorpholino doxorubicin. The arrow indicates the $C_{13}$ carbonyl moiety for linker attachment.

Figure 2 shows the Lewis acid catalyzed (open circles) and uncatalyzed (filled circles) reactions of 4-hydrazino sulfonylbenzoic acid with cyanomorpholino doxorubicin at 25 ° C. Also shown is the catalyzed reaction at 4 ° C (half-filled circles).

Figure 3 shows the Lewis acid catalyzed (open circles) and uncatalyzed (filled circles) reactions of 4-hydrazino sulfonylbenzoic acid with morpholino doxorubicin at 35 ° C.

Figure 4 shows the reaction rates catalyzed by various Lewis acids of morpholino doxorubicin with 4-hydrazino sulfonyl benzoic acid. The acids are: ■ uncatalyzed;♦ lithium chloride; □ $BF_3$ etherate; ⍖ is tosyl chloride [HCl];◊ toluene sulfonic acid.

Figure 5 shows the immunoreactivity of: an unconjugated antibody (open squares); a morpholino anthracycline-antibody conjugate on A-375 target cells (filled squares); an unconjugated non-specific antibody on A-375 cells (open triangles); and a morpholino anthracycline-antibody conjugate on UCLA-P3 non-target cells (filled triangles).

Figure 6 shows the cytotoxic effects of the morpholino anthracycline derivatives morpholino doxorubicin (open squares) and a morpholino doxorubicin-antibody conjugate (filled squares) on A-375 cells.

Figure 7 shows the cytotoxic effects of morpholino anthracyclines-antibody conjugates on A-375 target cells (open squares) and UCLA-P3 non-target cells (filled squares).

Figure 8 shows the efficacy of morpholino anthracyclines-antibody conjugates on suppressing tumor growth in nude mice. Open squares represent antibody or saline control; filled squares represent free drug; open triangles represent a mixture of drug and antibody; the filled triangles represent tumor growth from animals injected with MRA conjugates having an initial tumor size of 4 $mm^2$; X represents animals injected with MRA conjugates two days after inoculation with tumor cells.

## DETAILED DESCRIPTION OF THE INVENTION

This invention is directed to simple and efficient methods of producing novel morpholino anthracycline compounds and to methods of coupling these morpholino anthracycline compounds to antibodies. An advantage of such conjugates is that morpholino anthracycline compounds are highly cytotoxic and coupling of the drug to an antibody is an effective way to specifically target any desired cell type to which there is a specific antibody. Thus, the invention is applicable for therapeutic use against the growth of a variety of cell types such as cancer.

In one embodiment, the morpholino anthracycline compound, cyanomorpholino doxorubicin (CN-MRA), is attached to a linker molecule through the $C_{13}$ carbonyl moiety on the doxorubicin component. The linker molecule, hydrazino sulfonylbenzoic acid, is an acid labile heterobifunctional linker with a hydrazine moiety on one end and a carboxylic acid moiety on the other end. Heterobifunctional linkers are preferable because they provide specificity to the reaction and thereby eliminate unwanted side products in the coupling reactions. The acid lability of the linker regenerates the free drug which can influence its potency; however, linkers cleavable by other means can work as well. The hydrazine end of the linker reacts with the carbonyl moiety to form a covalent bond. Hydrazides and oximes can also react with the carbonyl moiety to form a covalent bond. The reaction is catalyzed by a Lewis acid such as $BF_3$. The resultant morpholino anthracycline-linker compounds can be coupled to target cell binding proteins such as antibodies to produce morpholino anthracycline-antibody conjugates.

As used herein, the term "morpholino anthracycline" or "morpholino anthracycline compound" refers to a cytotoxic drug of the morpholino class of compounds. This term includes derivatives and analogs which contain a carbonyl moiety on the $C_{13}$ substituent of the cyclohexane ring of, for example, the doxorubicin or danorubicin moiety. An example of a morpholino anthracycline compound is cyanomorpholino doxorubicin and is shown in Figure 1. The arrow indicates the carbonyl moiety on the $C_{13}$ substituent. The term also includes those derivatives and analogs of morpholino anthracycline compounds with chemically equivalent moieties at positions other than the $C_{13}$ carbonyl moiety.

As used herein, the term "reactive group" refers to a chemical moiety which exhibits bond formation activity. Coupling a molecule through a reactive group on a second molecule results in bond formation between the two molecules. The reacting moiety on the first molecule being coupled is therefore also a reactive group. Reactive groups can be, for example, hydrazines, hydrazides, oximes, primary amines, carboxylic acids, hydroxyls and isocyanates. Other reactive groups known to one skilled in the art can also be included. The term is also meant to include bond formation between two reactive groups regardless of prior activation to achieve bond formation.

As used herein, the term "linker" or "linker molecule" refers to a molecule which can form a bridge-like structure between a morpholino anthracycline compound and an antibody. The linker can be used, for example, as a means for coupling a morpholino anthracycline compound to an antibody. Such linkers can consist of one or more atoms, such as activating moieties, but are preferably between 6 and 30 atoms in length, more preferably between 8 and 12 atoms. The term includes both bifunctional, and more preferably, heterobifunctional linkers, both of which can be acid labile. Both types of linkers contain reactive groups on each end of the molecule for use in coupling morpholino anthracycline compounds to an antibody. Bifunctional linkers have two similar chemical reactive groups. Heterobifunctional linkers, on the other hand, have different reactive groups at each end. Heterobifunctionality is beneficial because it reduces crosslinking and the production of unwanted side products in the coupling reaction. Heterobifunctional linkers include, for example, hydrocarbons of about seven to twenty carbon atoms in length. Preferably, a carboxylic acid moiety is included as one of the chemical reactive groups. The other chemical reactive group will contain one of a variety of chemical moieties which can be, for example, a hydrazine, hydrazide or oxime moiety.

Peptide linkers are also included which have, for example, the structure: R-Peptide-$R_1$-COOH. R corresponds to the morpholino anthracycline-reactive end and includes, for example, chemical moieties such as hydrazines, hydrazides and oximes. $R_1$ corresponds to the antibody-reactive end and can

include, for example, structures such as maleic acid, succinic acid, citraconic acid, diglyconic acid and dimethylmaleic acid. The peptide can be of any length and sequence but is preferably about three to four amino acids in length. The sequence is preferably Ala-Ala-Ala, Ala-Leu-Ala-Leu, Leu-Ala-Leu-Ala, Ser-Ser-Ser, Gly-Gly-Gly-Leu, Gly-Phe-Leu-Gly or Gly-Phe-Phe-Leu.

As used herein, the term "coupled" refers to the two molecules joined together. The two molecules can be, for example, a morpholino anthracycline compound and a linker molecule. Morpholino anthracycline-linker compounds and antibodies can also be joined together by coupling to produce a morpholino anthracycline-antibody conjugate. Therefore, "coupling reaction" as used herein refers to the chemical reaction for joining two molecules together.

As used herein, the term "antibody" refers to a antibody which exhibits selective binding to a target cell. The antibody can be, for example, polyclonal or monoclonal. The term is also meant to include fragments of antibodies such as Fab' or $(Fab)_2$ fragments. "Functional fragments" as used herein refers to fragments which exhibit the selective binding properties to a target cell as that of the intact molecule. Selective binding includes binding specificity and affinity of the antibody for the target cell. Binding is typically to a cell surface molecule such as a protein antigen. Binding to other cell surface molecules can include antigens such as carbohydrate, lipid, inorganic molecules such as phosphorylated and sulphated moieties, as well as polypeptide and peptide so long as the antibody exhibits selective binding for these antigens. The target cell can be any cell or population of cells which contain a cell surface molecule capable of being bound to the antibody. This includes cells in culture as well as cells within an organism.

The invention provides for morpholino anthracyclines coupled to linker molecules and methods for producing such compounds which have the structure M-L where M is a morpholino anthracycline compound and L is a linker molecule which is attached to the $C_{13}$ substituent of the cyclohexane ring of M. Morpholino anthracycline compounds contain a carbonyl moiety on the $C_{13}$ substituent of the terminal cyclohexane ring. This carbonyl moiety can be advantageously used to attach a linker molecule such as hydrazino sulfonylbenzoic acid (HSB). Morpholino anthracycline compounds with chemically equivalent carbonyl moieties can also be used. Other hydrazine containing linkers as well as hydrazide and oxime containing linkers can also be used as well.

HSB or any of the above linker molecules can be coupled to morpholino anthracycline compounds by reacting the morpholino anthracycline and linker with a Lewis acid catalyst. An example of a Lewis acid useful for catalysis is $BF_3$ although other Lewis acids can be used as well. The reaction of a morpholino anthracycline compound such as cyanomorpholino doxorubicin with HSB is as follows:

$$\text{M-C=O} + \text{H}_2\text{N-NH-X-C}_6\text{H}_4\text{-CO}_2\text{H} \xrightarrow{BF_3} \text{M-C=N-NH-X-C}_6\text{H}_4\text{-CO}_2\text{H} + \text{H}_2\text{O}$$

where M-C=O represents the $C_{13}$ carbonyl moiety on morpholino anthracycline compounds and where X within the linker is a $SO_2$ group for HSB, a carbonyl group for other linkers or nil (i.e., a direct bond to the phenyl ring).

The morpholino anthracycline-linker compounds can further be coupled to, for example, an antibody to produce morpholino anthracycline-antibody conjugates. The antibody can be polyclonal or monoclonal. The antibody can also be functional fragments such as Fab or $(Fab)_2$. The free end of the linker molecule, for example, the carboxylic acid moiety of HSB, can be activated by methods known to one skilled in the art. The activated moiety can then be coupled to free amino groups on proteins, such as antibodies, to produce morpholino anthracycline-antibody conjugates. Activation typically does not change greatly the chemical specificity of the chemical moiety, but instead, allows the chemical moiety to be reacted faster, easier and often with the production of fewer side products. Activation of the carboxylic acid moiety with reagents such as carbodiimide, N-hydroxysuccimide, N-ethoxycarbonyl-2-ethoxy-1, 2-dihydroquinoline (EEDQ) or isobutyl chloroformate, will allow rapid and efficient coupling of the activated carboxylic acid moiety with a primary amine to form an amide bond. Other activating reagents include, for example, isocyanates, thioisocyanates, azides and diazo-halides. The activating reagent used will depend on the particular chemical moiety involved and on the desired bond to be produced. These reactions are known to one skilled in the art and can be performed readily.

The extent of the coupling reactions should be such that the antibody retains functionality. Mild reaction conditions is one way to maintain antibody functionality. Such reaction conditions are standard procedures for one skilled in the art as well as the determination of the functionality of the resultant morpholino anthracycline conjugates. Additional

steps, such as binding the antibody to antigen prior to coupling for protection of the binding site, can also be used to ensure functionality of the morpholino anthracycline-antibody conjugates.

Since morpholino anthracyclines are extremely cytotoxic to all types of cells, the morpholino anthracycline conjugates can be used to specifically target morpholino anthracyclines to any cell type in which a specific antibody is available. Other proteins which show specific binding properties to cell antigens can also be coupled to the morpholino anthracycline-linker compounds. Targets can include, for example, viral or bacterial cells containing specific antigens as well as host cells containing specific antigens recognized by an antibody.

The morpholino anthracycline-antibody conjugates of the present invention can be used to deliver morpholino anthracycline compounds and to produce cytotoxic effects on target cells. For example, it is possible to use such conjugates to destroy a certain population of cells, such as T cells or B cells in an organism by coupling a morpholino anthracycline to an antibody which exhibits binding specificity to the desired population of T or B cells. Additionally, tumor cells can be targeted by coupling a morpholino anthracycline compound to an antibody which selectively binds the tumor cell. In a similar manner, a specific cell type can be purified from a contaminating population of cells in culture with a morpholino anthracycline conjugate in which the antibody has binding specificity for the contaminant cell type. Once the antibody is bound to the target cell and internalized by cellular processes, regeneration of the free drug occurs due to the acid lability of linkers such as HSB. Therefore, the invention provides a method of delivering a morpholino anthracycline conjugate to immunoreactive cells and for remitting tumor growth of such cells. An example is the purging of bone marrow contaminated with tumor cells by mixing the bone marrow with an antibody-morpholino compound.

The following Examples are intended to illustrate, but not limit, the invention.

## EXAMPLE I

### Coupling of Hydrazino Sulfonylbenzoic Acid to Morpholino Doxorubicin and Cyanomorpholino Doxorubicin

This example illustrates the coupling of hydrazino sulfonylbenzoic acid (HSB) linker molecules to morpholino doxorubicin (MRA) cyanomorpholino doxorubicin (CN-MRA) to produce a morpholino anthracycline-linker compound.

CN-MRA was coupled to HSB by mixing 100 µl of N-methylpyrrolidone (NMP) containing 2.89 mg (4.51 x $10^{-6}$ moles) with 20 µl of HSB stock (5.3 mg HSB/100 µl NMP). The mixture was reacted at 25°C with or without the addition of 5 µl of $BF_3$ etherate/100 µl NMP as a catalyst. An aliquot was also reacted at 4°C using $BF_3$ as a catalyst. The yield of morpholino anthracycline-linker compound, designated CN-MRA-HSB, was followed over time by HPLC analysis (16-fold dilution in methanol, 5 µl injected) and is shown in Figure 2 as the percentage of product conversion. Open and closed circles designate the $BF_3$ catalyzed and uncatalyzed reactions at 25°C respectively. Half-filled circles designate the $BF_3$ catalyzed reaction at 4°C.

CN-MRA was also reacted as described above using a reaction temperature of 40°C instead of 25°C. HPLC analysis showed multiple product peaks characteristic of many decomposition products.

Using the above protocol for the coupling of HSB to CN-MRA, MRA was also coupled to HSB except that the reaction temperature was 35°C. The extent of product conversion to a morpholino anthracycline-linker compound, designated MRA-HSB, for $BF_3$ catalyzed (open circles) and uncatalyzed (closed circles) reactions are shown in Figure 3.

## EXAMPLE II

### Comparisons of Lewis Acids for Catalyzing the Coupling of Hydrazino Sulfonylbenzoic Acid to Cyanomorpholino Doxorubicin

MRA (3.57 mg, 1.56 x $10^{-5}$ moles) in 100 µl DMAC was mixed with 42 µl HSB stock (1.6 x $10^{-5}$ moles) in DMAC. Then 20 µl aliquots were prepared and to each separate sample was added 2 µl of Lewis acid in DMAC as shown in Figure 4. The mixtures were agitated in a constant temperature bath at 25°C and 1 µl test samples were removed for HPLC analysis of the reaction rates. The results are shown in Figure 4 where ■ is an uncatalyzed reaction; ♦ is catalyzed with LiCl; □ is $BF_3$ etherate; ⍖ is tosyl chloride [HCl];◊ is toluene sulfonic acid.

## EXAMPLE III

### Coupling of a CN-MRA-HSB Morpholino Anthracycline-Linker Compound to an Antibody

This example illustrates the coupling of a CN-MRA-HSB morpholino anthracycline-linker compound to an antibody to produce a morpholino anthracycline-antibody conjugate.

The cyanomorpholino doxorubicin-HSB derivative of Example I was activated by adding 10 µl

each of a stock solution of N-hydroxysuccinimide (NHS) and ECDI (5 x $10^{-7}$ moles/µl) and stirring at 25°C in the dark for 1 hour followed by reaction at 4°C for 18 hours. This solution of CN-MRA-HSB, produced as described in Example I, was added in 40 µl portions to 3 mls (36 mg = 2.32 x $10^{-7}$ moles) of the monoclonal antibody 9.2.27 which had previously been adjusted to pH 8.0. The total amount of CN-MRH-HSB conjugate was 3.7 mg (5.78 x $10^{-6}$ moles) which was a 25-fold molar excess. The 40 µl additions were made at 10 minute intervals. A clear reddish solution was observed throughout the addition of CN-MRA-HSB. The pH was maintained at pH 8.0 after each addition by adding 5 µl of 1 N NaOH.

After 80 µl of CN-MRA-HSB was added (3.55 x $10^{-6}$ moles), a first aliquot of 100 µl was removed and G-50 column purified using PBS buffer. Some turbidity was subsequently observed in the reaction after 30 minutes and a second aliquot of 150 µl was purified. The remainder of the reaction mixture was centrifuged at 13,000 rpm for 5 minutes to remove the precipitated material and purified by gel filtration chromatography on Sepharose™ G-50 using PBS buffer (50 mM).

For the second aliquot and the G-50 purified solution, absorbance measurements were taken at 280 nm and at 485 nm to determine the extent of coupling. These measurements were used to calculate the molar ratio of antibody to drug using the following equation: Molar Ratio = $A_{480}$/13 x $10^6$/mg/ml antibody/155,000 where the mg/ml antibody was calculated using the formula:

$$mg/ml = \frac{A_{280} - (0.72 \times A_{480})}{1.4}$$

where the molar extinction coefficient at 480 nm for CN-MRA is estimated to be 13,000.

EXAMPLE IV

Immunoreactivity of Morpholino Anthracycline-Antibody Conjugates

To assess whether the morpholino anthracycline-antibody conjugates (products from Example III) retained binding activity and antigen specificity, an ELISA was performed on A-375 (target) and UCLA-P3 (non-target) cell lines (Figure 4). Unlike the UCLA-P3 cell line, the A-375 line expresses antigens which are specifically recognized by the monoclonal antibody 9.2.27.

Appropriate cell lines that were in logarithmic growth phase were harvested using 1 mM EDTA. The cells were centrifuged at 1000 x g and rinsed 2 times with 1 x PBS. The cell pellet was resuspended in 1 x PBS in a volume to equal 1 x $10^6$ cells/ml. Using a 12 channel micropipettor, cells were platted out in 50 µl/well volume to equal 5 x $10^4$ cells/well in flexible 96 well plates (Falcon #3912, Becton Dickinson & Co., Lincoln Park, NJ). The 96 well plates were placed in a dry 37°C incubator for 3 to 5 days until the wells were completely dry. The new plates were tested with purified monoclonal antibody standards before using in specificity assays.

The above prepared dry cell plates (both target and non-target cells) were coated with 200 µl of 0.5% BSA in PBS (0.5% BSA/PBS). The plates were placed on a shaker for 1 hour at room temperature and then washed with 0.5% BSA/PBS in the following manner. For the first wash, 300 µl of 0.5% BSA/PBS was added and immediately flicked out followed by slapping dry on a teri towel. For the remaining washes, 300 µl of 0.5% BSA/PBS was added and allowed to incubate at room temperature for 3-5 minutes before flicking dry. Following the final wash, the plates were slapped dry on a teri towel.

To the above prepared and washed plates, standards and samples were added in 100 µl volumes/well starting at 10 µg/ml with serial dilutions down to 1 pg/ml. The samples were diluted in 0.5% BSA/PBS and were incubated for 1 hour at room temperature while shaking. Following incubation, the plates were washed as described above and 100 µl/well of secondary antibody (goat anti-mouse coupled to horseradish peroxidase (Bio-Rad Laboratories, Richmond, CA)) was added at 1:4000 dilution in 0.5% BSA/PBS. The antibody was allowed to bind for 30 minutes while shaking at room temperature. The plates were again washed as described above and peroxidase staining was developed for 5-20 minutes with shaking by addition of 100 µl/well of OPD/Phos-citrate buffer (40 ml of phosphate-citrate buffer, 12 mg o-phenylene-diamine, 6 µl $H_2O_2$). Reactions were quenched by addition of 50 µl/well of 4N sulfuric acid and read at 490 nm on a titertek Multiskan MCC/340 elisa reader (Flow Laboratories, McLean, VA).

The results shown in Figure 5 demonstrated that both the unconjugated (open squares) and conjugated (filled squares) 9.2.27 monoclonal antibodies specifically bound to the A-375 cell line compared to control antibody KS 1/4 (open triangles). Moreover, the CN-MRA-HSB-Antibody conjugate retained nearly all its functional activity (compare open and filled squares) demonstrating that the chemical procedures used to couple CN-MRA to a target cell binding protein did not have any detrimental effects on antigen binding and specificity.

To ensure that coupling of CN-MRA to an antibody did not adversely affect its cytotoxicity, A-375 cells were treated as above with serial dilutions of a morpholino anthracycline-antibody conjugate (MRA-antibody conjugate coupled as described in Examples I and II) and the uncoupled morpholino anthracycline drug alone. Figure 6 shows the results obtained. Identical cytoxicity was observed for both the morpholino anthracycline alone (open squares) and the morpholino anthracycline-antibody conjugate (filled squares) These results demonstrated that the observed cytotoxic effects were not affected by the coupled antibody.

EXAMPLE V

Cytotoxicity of Morpholino Anthracycline-Antibody Conjugates on Cultured Cells

This example illustrates the specific cytotoxic effects of morpholino anthracycline-antibody conjugates on antigen- containing target cells compared to non-target cells.

A-375 target and UCLA-P3 non-target cell lines were cultured in RPMI tissue culture media supplemented with 10% fetal bovine serum (FBS). The cell lines ($1x10^4$/ml) were plated in 100 $\mu$l volumes and incubated with serial dilutions of the morpholino anthracycline-antibody conjugate for 2 hours (open squares, A-375 target cells; filled squares, UCLA-P3 non-target cells). Thereafter, the plates were washed three times in tissue culture media and incubated overnight. Each well received 1 $\mu$Ci of $^3$H-thymidine and after 16 hour incubation, the cells were harvested onto glass fiber filters with a PHD cell harvester (Cambridge Technology Inc., Cambridge, MA). The filters were placed in Ecolume scintillation solution (International Chemical and Nuclear Pharmapseuticals Inc., Irvine, CA) and were counted in a scintillation counter. $^3$H-Thymidine incorporation, as percentage of cell survival, is used to express cytotoxicity. The results, shown in figure 7, demonstrated specific cytotoxic effects on A-375 target cells using CN-MRA-antibody conjugates.

EXAMPLE VI

Cytotoxicity of Morpholino Anthracycline-Antibody Conjugates in Nude Mice

This example illustrates the efficacy of morpholino anthracycline-antibody conjugates in suppressing tumor growth in nude mice.

Nude mice Balb/c (Nu/Nu) (Imdyne Inc., San Diego, CA) were injected with M-21 tumor cells at a density of 2 x $10^6$/ml. The mice were kept for 7 days prior to injection of immunoconjugates. Mice were injected with either saline or 500 $\mu$g/200 $\mu$l of unconjugated 9.2.27 control antibody (open squares); MRA alone (filled squares); a mixture of MRA plus 500 $\mu$g/200 $\mu$l of unconjugated 9.2.27 antibody (open triangles); MRA conjugates injected two days after inoculation (X); or MRA conjugates injected into mice with initial tumor sizes of 4 mm$^2$. The total amount of MRA given per mouse was 2 $\mu$g in two injections.

The studies were carried out in athymic mice bearing palpable, measurable subcutaneous (s.c.) tumors of M-21 cell line. All animals had palpable, measurable tumors of about 2 x 2 mm or 4 mm$^2$ at the time therapy was started. Each individual experiment contained ten animals in each of six arms including a PBS control, antibody alone, drug alone, the immunoconjugate specific for antigen present on M-21, the immunoconjugate not specific for antigen present on M-21, and a mixture of antibody with drug. Each animal received an i.p. injection once a week for two weeks. Animals were observed daily for general well-being and survival. Maximum cross-sectioned diameters were measured every third day using calipers. The product of the two cross-section diameters was determined and recorded as tumor size. The results are graphed in Figure 8. Tumors of those mice which received the control injections grew to sizes in the range of 500 to 1500 mm$^3$. Those mice which received injections of MRA-antibody conjugates had tumors whose growth rate was significantly suppressed to a size of 50-200 mm$^3$ after two weeks of treatment.

Although the invention has been described with reference to the presently-preferred embodiment, it should be understood that various modifications can be made without departing from the spirit of the invention. Accordingly, the invention is limited only by the following claims.

## Claims

1. A morpholino anthracycline-linker compound comprising the structure M-L, wherein M is a morpholino anthracycline compound and L is a linker molecule which is attached to the carbonyl moiety on the $C_{13}$ substituent of the cyclohexane ring of M.

2. The composition of claim 1, wherein M is cyanomorpholino doxorubicin.

3. The composition of claim 1, wherein L is a linker comprising a reactive group selected from the group consisting of hydrazine, hydrazide and oxime.

4. The composition of claim 3, wherein L is cleavable and regenerates free M.

**5.** The composition of claim 3, wherein L is heterobifunctional.

**6.** The composition of claim 3, wherein L is hydrazino sulfonylbenzoic acid.

**7.** A morpholino anthracycline-antibody conjugate comprising the structure M-L-Ab, wherein Ab is an antibody or functional fragment thereof, M is a morpholino anthracycline compound and L is a linker molecule which is attached to the carbonyl moiety on the $C_{13}$ substituent of the terminal cyclohexane ring of M.

**8.** The composition of claim 7, wherein M is cyanomorpholino doxorubicin.

**9.** The composition of claim 7, wherein L is a linker comprising a reactive group selected from the group consisting of hydrazine, hydrazide and oxime.

**10.** The composition of claim 9, wherein L is cleavable and regenerates free M.

**11.** The composition of claim 9, wherein L is heterobifunctional.

**12.** The composition of claim 9, wherein L is hydrazino sulfonylbenzoic acid.

**13.** The composition of claim 7, wherein Ab is a monoclonal antibody.

**14.** A method of preparing a morpholino anthracycline compound coupled to a linker molecule comprising combining a Lewis acid with a morpholino anthracycline compound and a linker molecule at a temperature sufficient to release $H_2O$, the linker molecule comprising a reactive group selected from the group consisting of hydrazines, hydrazides, or oximes to produce a morpholino anthracycline-linker compound.

**15.** The method of claim 14, wherein the morpholino anthracycline compound is cyanomorpholino doxorubicin.

**16.** The method of claim 14, wherein the Lewis acid is $BF_3$.

**17.** The method of claim 14, wherein the linker molecule is heterobifunctional.

**18.** The method of claim 14, wherein the morpholino anthracycline-linker compound is further coupled to an antibody, or functional fragment thereof, to produce a morpholino anthracycline-antibody conjugate.

**19.** The method of claim 18, wherein the antibody is a monoclonal antibody.

**20.** Use of a morpholino anthracycline-antibody conjugate for the preparation of a medicament having selective immunoreactivity for cells.

**21.** Use of claim 20 for the preparation of a medicament for remitting tumor growth.

Figure 1

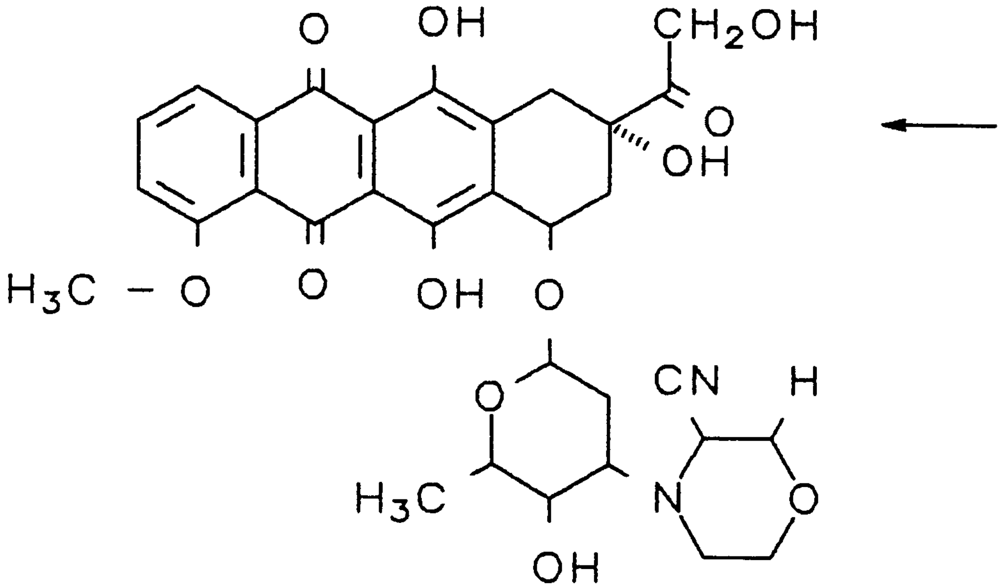

FIGURE 2

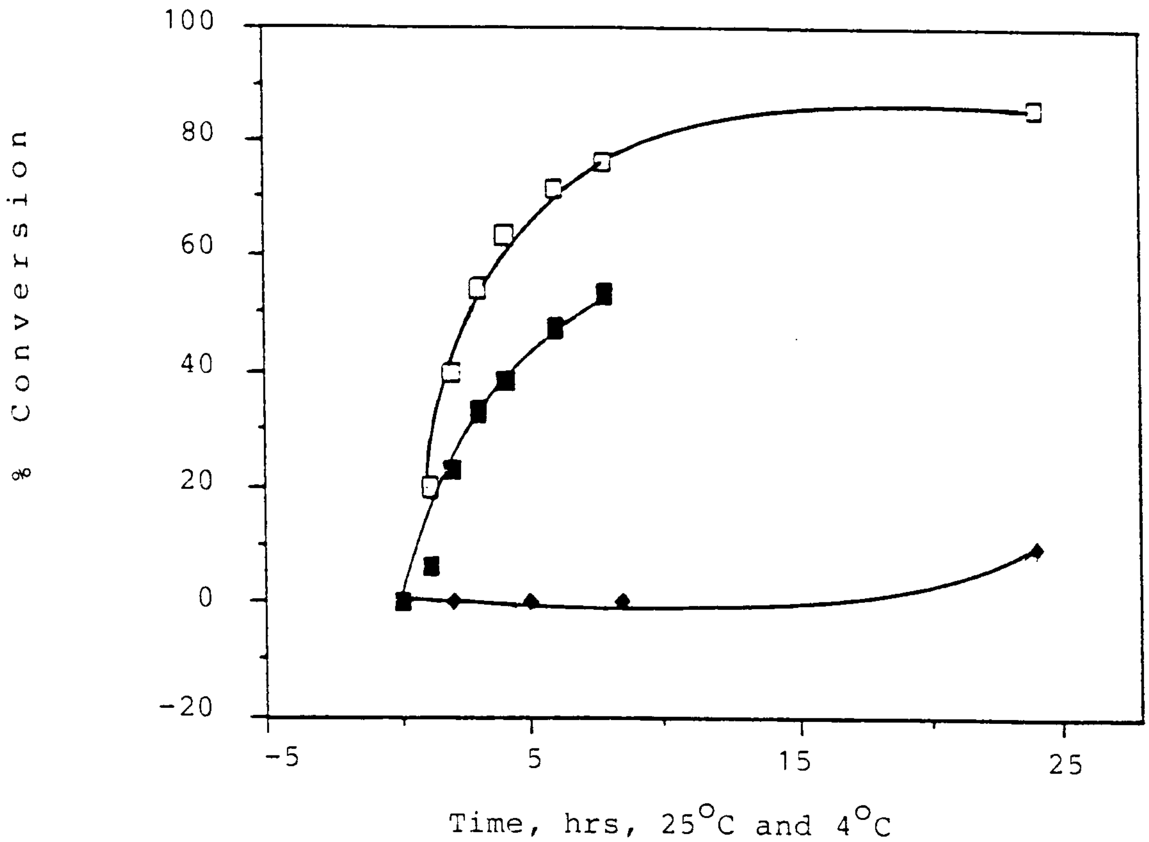

FIGURE 3

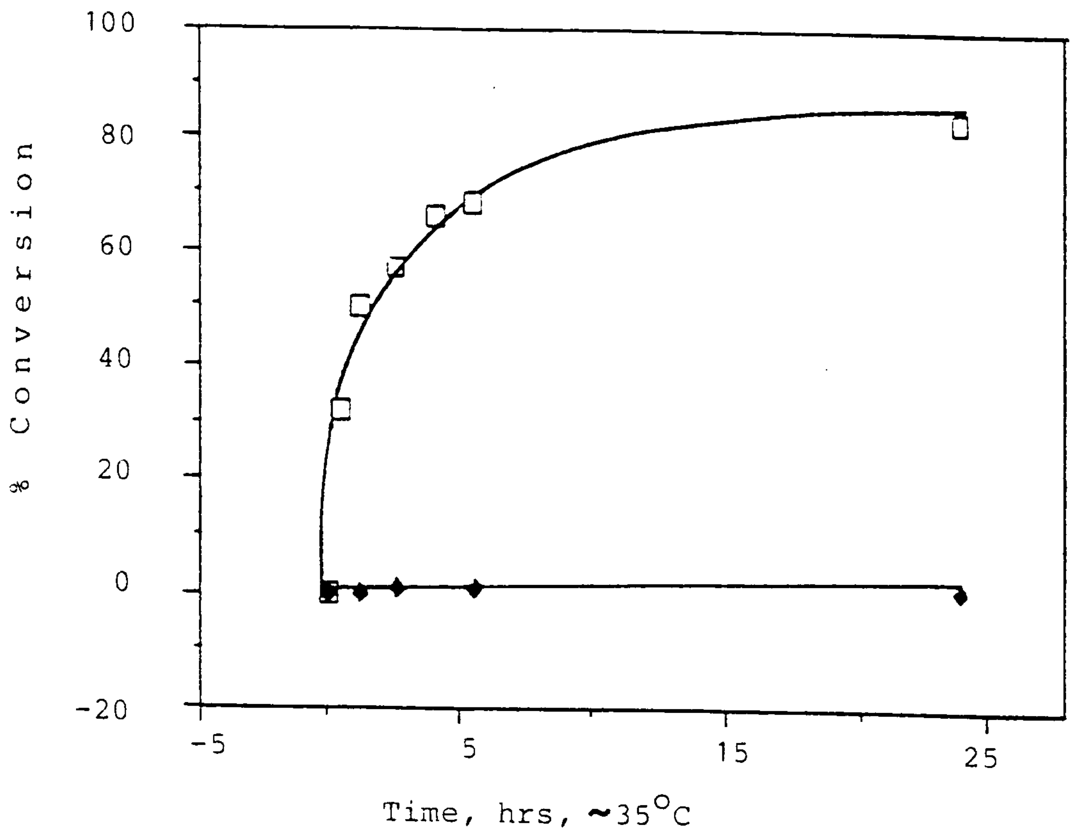

FIGURE 4

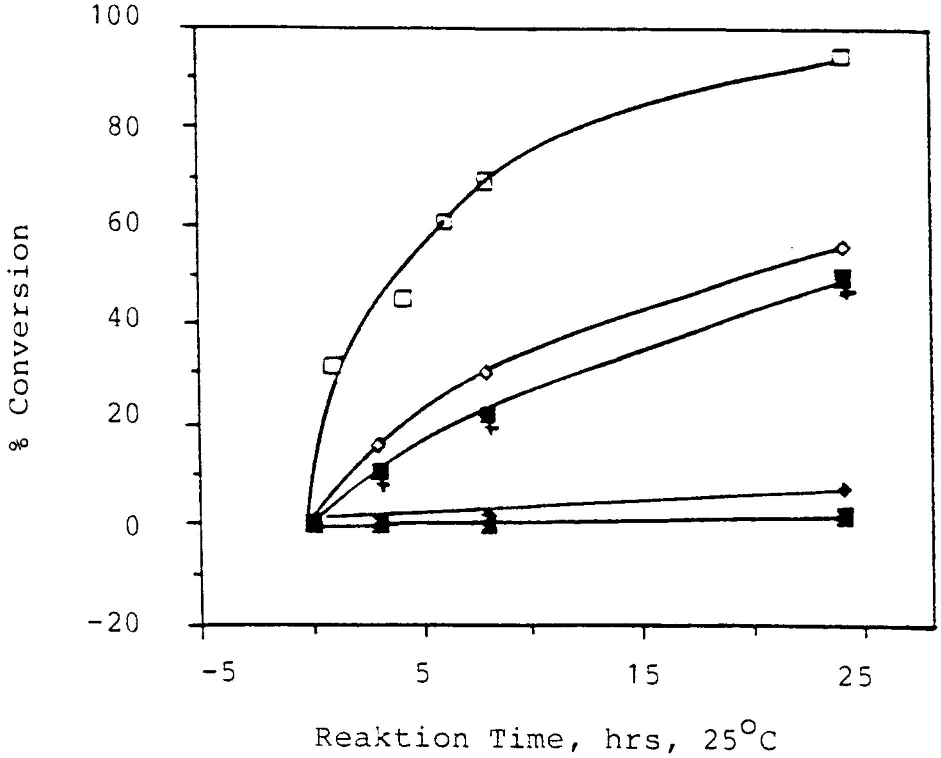


Reaktion Rates for the Lewis Acid Catalysed
Reaktion of Morpholinodoxorubicin with
4-hydrazino sulfonyl benzoic Acid

FIGURE 5

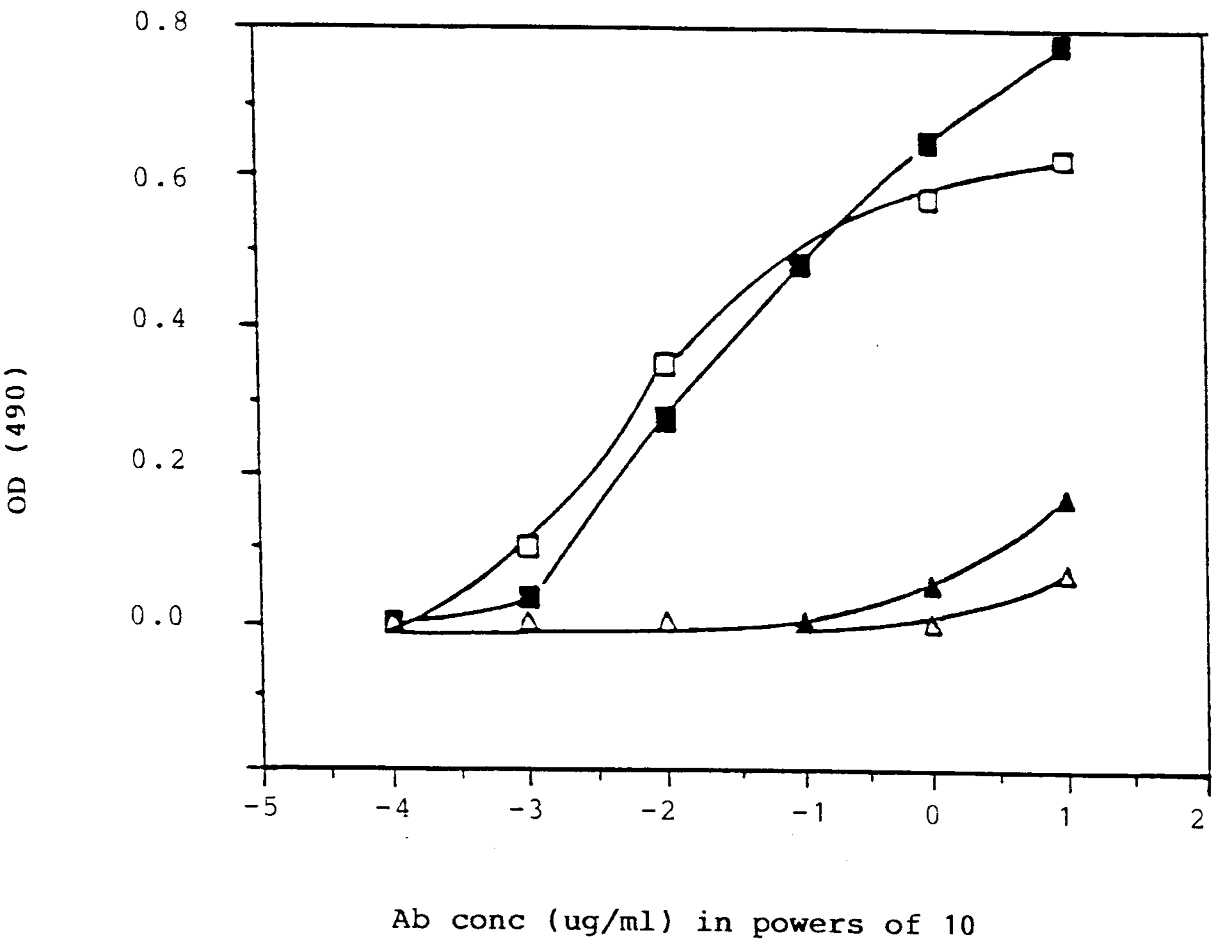

FIGURE 6

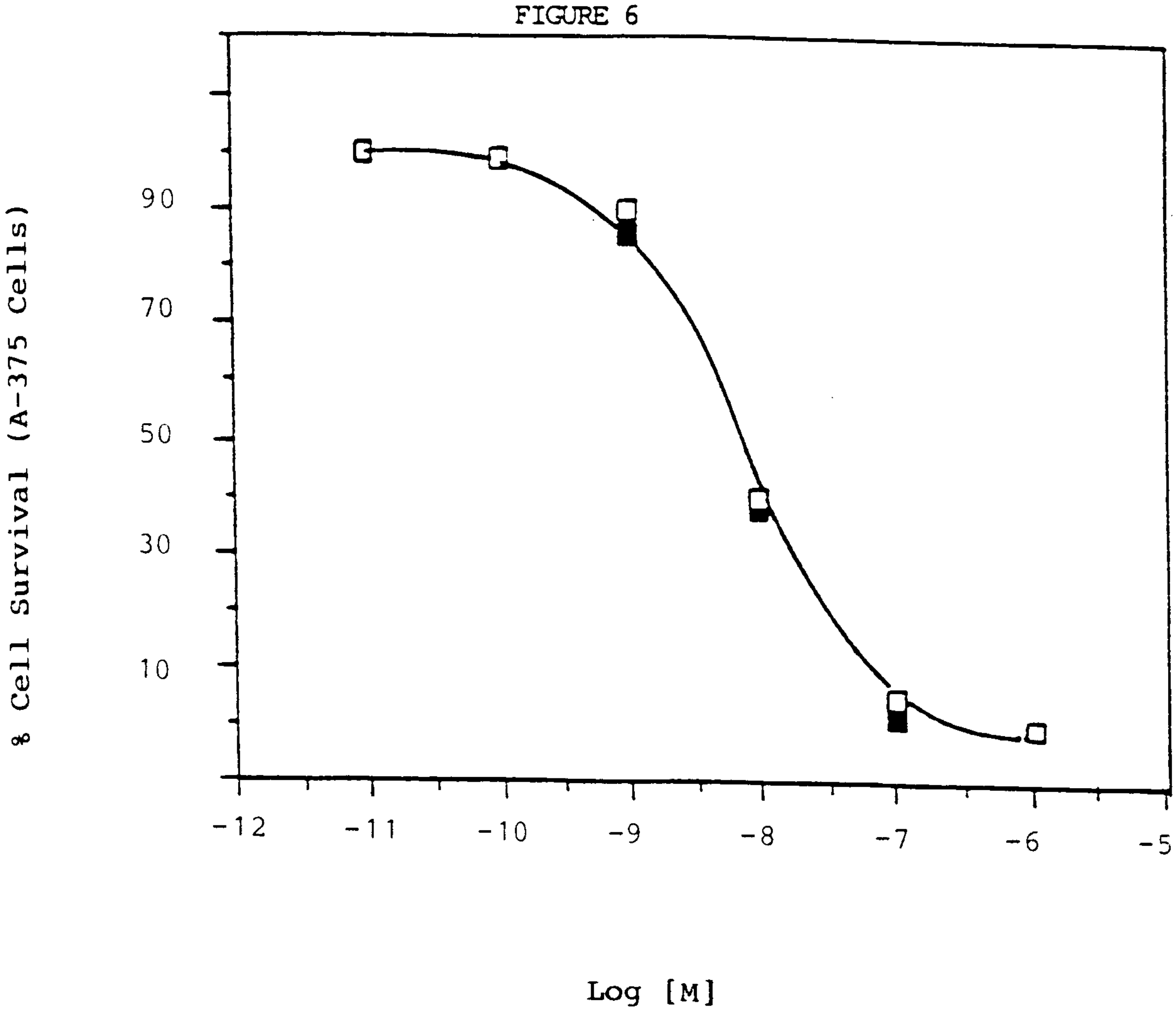

FIGURE 7

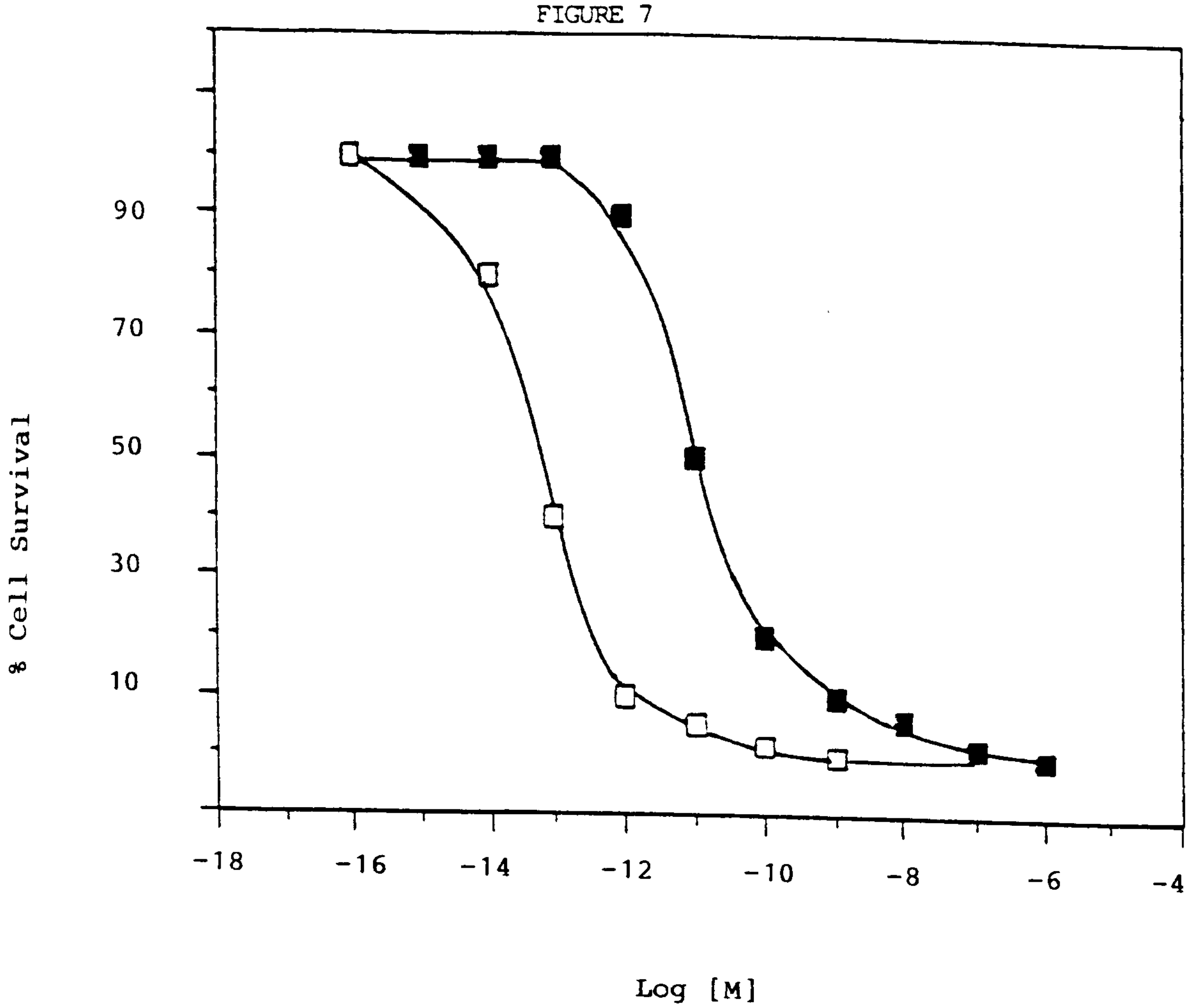

FIGURE 8

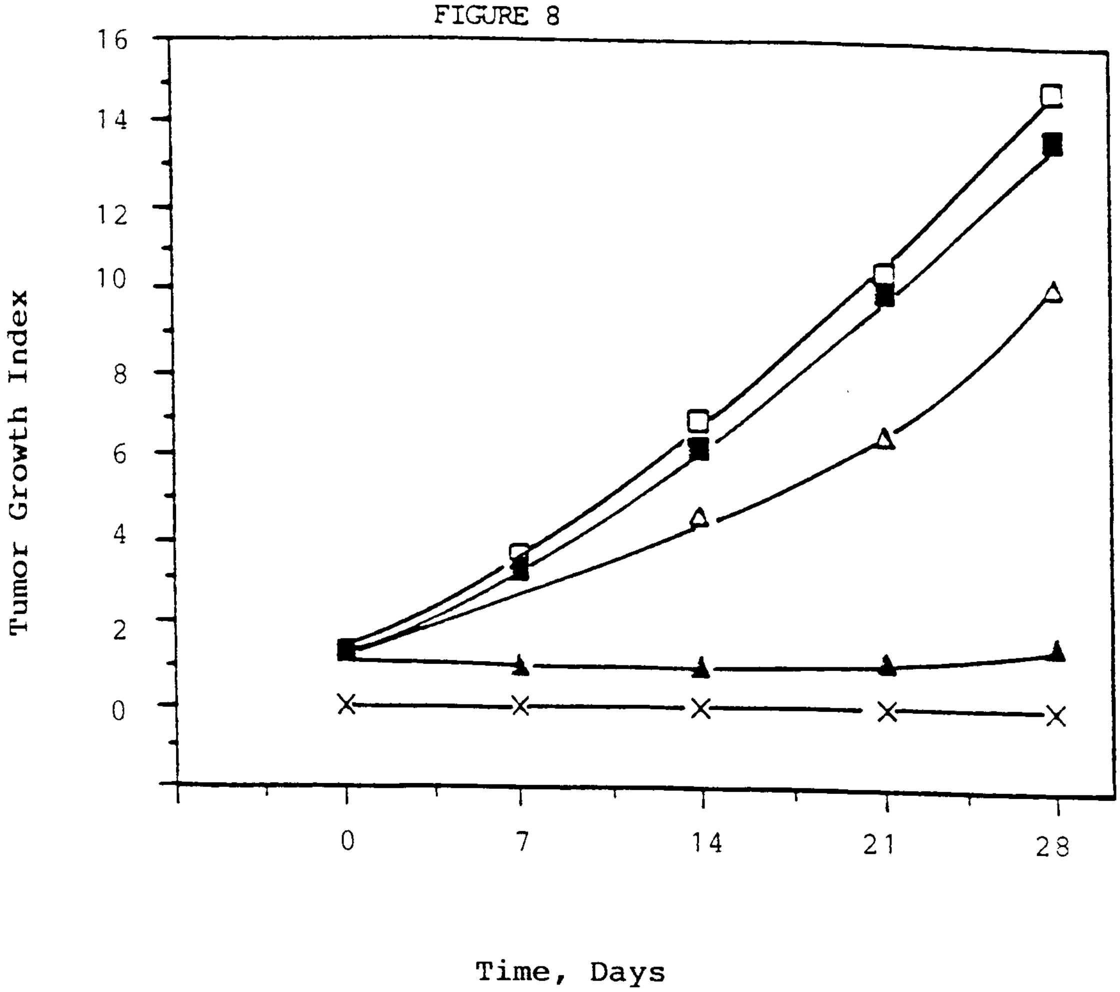

European Patent Office

# EUROPEAN SEARCH REPORT

Application Number

EP 91114725.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A - 0 328 147 (BRISTOL-MYERS COMPANY) * Abstract; claims 1,4,6,9, 11,12,16,22,24,26 * | 1-5, 7-11, 13, 20,21 | A 61 K 47/48<br>A 61 K 39/395<br>//A 61 K 31/71<br>A 61 K 31/535 |
| A | * Claims 1,4,6,9,11,12, 16,19 * | 6,14-19 | |
| P,X | EP - A - 0 398 305 (BRISTOL-MYERS SQUIBB COMPANY) * Abstract; claims 1,3,7, 15-17; page 7, line 34 - page 8, line 36 * | 1-5, 7-11, 13, 20,21 | |

TECHNICAL FIELDS SEARCHED (Int. Cl.5)

A 61 K 47/00
A 61 K 39/00
A 61 K 31/00

The present search report has been drawn up for all claims

| Place of search | Date of completion of the search | Examiner |
|---|---|---|
| VIENNA | 22-11-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document